**Europäisches Patentamt**

**European Patent Office**

**Office européen des brevets**

(19)

(11) Veröffentlichungsnummer: **0 146 006**
**B1**

(12) # EUROPÄISCHE PATENTSCHRIFT

(45) Veröffentlichungstag der Patentschrift:
**16.09.87**

(51) Int. Cl.⁴: **A 61 B 6/04**

(21) Anmeldenummer: **84114165.8**

(22) Anmeldetag: **23.11.84**

(54) **Röntgendiagnostikgerät mit einem Kipptisch.**

(30) Priorität: **05.12.83 DE 3343877**

(43) Veröffentlichungstag der Anmeldung:
**26.06.85 Patentblatt 85/26**

(45) Bekanntmachung des Hinweises auf die Patenterteilung:
**16.09.87 Patentblatt 87/38**

(84) Benannte Vertragsstaaten:
**DE FR**

(56) Entgegenhaltungen:
**DE - C - 393 350**
**GB - A - 678 040**
**US - A - 2 534 623**
**US - A - 3 525 308**

(73) Patentinhaber: **Siemens Aktiengesellschaft Berlin und München, Wittelsbacherplatz 2, D-8000 München 2 (DE)**

(72) Erfinder: **Brendl, Rudolf, Leipzigerstrasse 49 1/2, D-8520 Erlangen (DE)**
Erfinder: **Hahn, Alfred, Bunsenstrasse 64, D-8520 Erlangen (DE)**
Erfinder: **Weiss, Karl, Im Föhrenwald 23, D-8520 Buckenhof (DE)**

ACTORUM AG

# Beschreibung

Die Erfindung betrifft ein Röntgendiagnostikgerät gemäss dem Oberbegriff des Patentanspruches 1.

Bei bekannten Röntgendiagnostikgeräten dieser Art ist der Kipptisch durch verschiedenartige Führungsmittel, z.B. Kurven- oder Getriebeführungen, in der Weise geführt, dass die erforderlichen Tischlagen, nähmlich auf der einen Seite der horizontalen Lage von 90° und auf der anderen Seite von beispielsweise 30° zur Horizontalen erreichbar sind. Die bekannten Führungsmittel, die insbesondere eine Längsverschiebung des Kipptisches mit seinem Gestell bei seiner Aufrichtung aus der Horizontallage bewirken, sind dabei relativ kompliziert aufgebaut.

In der US-A-3 525 308 ist ein Röntgendiagnostikgerät mit einem Kipptisch beschrieben, bei dem auf jeder Seite des Tischgestelles je eine Führungsbahn vorgesehen ist, in die ein Zapfen eingreift. Dadurch wird beim Kippen des Kipptisches aus der Horizontallage heraus dieser so angehoben, dass er nicht an dem Boden anstösst. In der Horizontallage ruht der Kipptisch auf jeder Seite nur auf je einem Zapfen.

In der GB-A-678 040 ist ein Röntgendiagnostikgerät mit einem Kipptisch beschrieben, bei dem der Kipptisch ein verzahntes Kreissegment aufweist, in das ein Zahnrad für die Kippung eingreift. Die Führung des Kipptisches erfolgt durch seitliche Zapfen, die in Schlitzen von vertikalen Stativen geführt sind.

Der Erfindung liegt die Aufgabe zugrunde, ein Röntgendiagnostikgerät gemäss dem Oberbegriff des Patentanspruches 1 so auszubilden, dass eine stabile Halterung des Kipptisches in allen Stellungen gewährleistet ist.

Diese Aufgabe ist erfindungsgemäss durch die im kennzeichnenden Teil des Patentanspruches 1 angegebene Ausbildung gelöst. Bei dem erfindungsgemässen Röntgendiagnostikgerät ruht das Tischgestell mit dem Kipptisch in der Horizontallage auf den beiden Führungszapfen. Wird es aus der Horizontallage nach einer Seite gekippt, so verlässt einer der Führungszapfen die zugeordnete Führungsschiene und der andere Führungszapfen bewirkt über seine Führungsschiene eine darartige Verstellung des Tischgestelles mit dem Kipptisch, dass es die gewünschten Kipplagen einnehmen kann, ohne am Boden anzustossen. Die Führungsmittel sind dabei sehr einfach aufgebaut, denn sie bestehen nur aus geradlinigen Führungsschienen, Führungszapfen und Antriebsmitteln.

Eine Entlastung des Antriebsrades und der Antriebsbahn von Auflagekräften wird erreicht, wenn neben dem Antriebsrad auf dessen Welle eine Tragrolle befestigt ist, die auf einer auf dem Tischgestell parallel zur Antriebsbahn verlaufenden Tragbahn abrollt. In diesem Fall nimmt das Traglager aus Tragrolle und Tragbahn die radialen Stützkräfte für das Tischgestell auf, so dass durch die zweckmässigerweise als Kette ausgebildete Antriebsbahn und das in diesem Fall als Kettenrad ausgebildete Antriebsrad nur Umfangskräfte zu übertragen sind.

Eine besonders stabile Führung ist gewährleistet, wenn symmetrisch zu beiden Geräteseiten je eine Antriebsbahn, ein Antriebsrad, eine Tragbahn, eine Tragrolle, zwei Führungsschienen und zwei Führungszapfen angeordnet sind. Damit das Tischgestell nicht unbeabsichtigt aus seinem Lager gehoben wird, können die Tragbahnen seitlich etwas über das Tischgestell vorstehen und von zu beiden Seiten des Tischgestells ortsfest angeordneten Sicherungshaken umgriffen werden.

Die Erfindung ist nachfolgend anhand eines in der Zeichnung dargestellten Ausführungsbeispieles näher erläutert. Es zeigen:

Fig. 1 ein Röntgendiagnostikgerät nach der Erfindung in einer Seitenansicht,

Fig. 2 und 3 das Röntgendiagnostikgerät gemäss Fig. 1 in verschiedenen Kipplagen, und

Fig. 4 eine Detailansicht des Gerätes gemäss den Figuren 1 bis 3 von hinten.

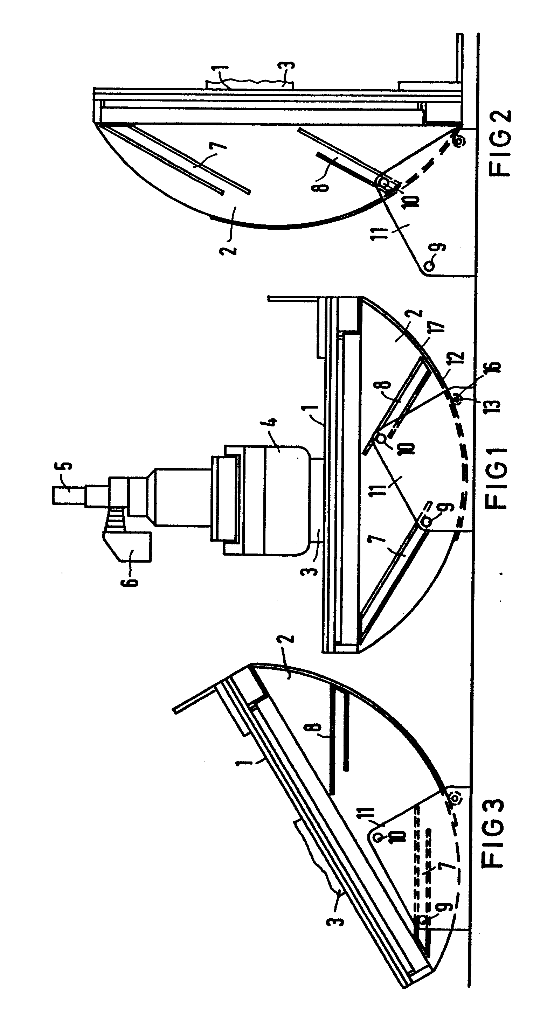

Das Gerät gemäss Fig. 1 weist einen Kipptisch 1 auf, der auf einem Tischgestell 2 motorisch längsverschiebbar gelagert ist. Unterhalb des Kipptisches 1 liegt eine nicht dargestellte Röntgenröhre, die an einem Träger 3 befestigt ist, der oberhalb des Kipptisches 1 einen Röntgenbildverstärker 4 mit einer nachgeschalteten Fernsehkamera 5 und einer Blattfilmkamera 6 trägt. In der in der Fig. 1 dargestellten Horizontallage des Kipptisches 1 liegt das Tischgestell 2 mit geradlinigen Führungsschienen, von denen in der Fig. 1 die Führungsschienen 7, 8 sichtbar sind, auf Führungszapfen auf, von denen in der Fig. 1 die Führungszapfen 9, 10 sichtbar sind. Die Führungszapfen 9, 10 usw. sind an ortsfesten, seitlich am Tischgestell 2 angeordneten Lagerteilen befestigt, von denen in der Fig. 1 das Lagerteil 11 sichtbar ist.

Das Tischgestell 2 ist an seiner Aussenseite mit zwei kreisförmigen Antriebsbahnen versehen, von denen in der Fig. 1 die Antriebsbahn 12 dargestellt ist. Mit der als Kette ausgebildeten Antriebsbahn 12 steht ein Zahnrad 13 in Eingriff, das durch einen Motor drehbar ist.

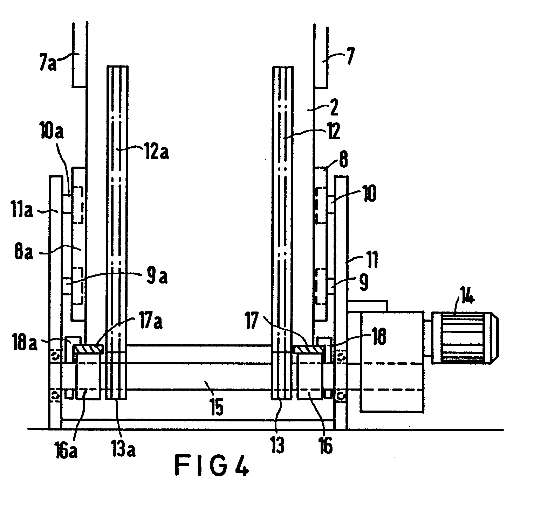

Die Fig. 4 zeigt, dass zu den Teilen 7 bis 13 symmetrisch am Gerät entsprechende Teile 7a bis 13a angeordnet sind. Das heisst, dass symmetrisch zu beiden Geräteseiten je eine Antriebsbahn 12, 12a, ein Antriebsrad 13, 13a, zwei Führungsschienen 7, 8, 7a, 8a und zwei Führungszapfen 9, 9a, 10, 10a vorhanden sind.

Aus der Fig. 4 geht auch hervor, dass auf der Welle 15, auf der die Antriebsräder 13, 13a befestigt sind, neben jedem Antriebsrad 13, 13a je eine Tragrolle 16, 16a befestigt ist, die auf einer auf dem Tischgestell 2 parallel zur Antriebsbahn 12, verlaufenden Tragbahn 17, 17a, die aus Flachmaterial besteht, abrollt.

Soll der Kipptisch 1 aus seiner Horizontallage, in der sich das Tischgestell 2 mit den Führungsschienen 7, 7a, 8, 8a auf den Führungszapfen 9, 9a, 10, 10a und mit den Tragrollen 16, 16a auf den Tragbahnen 17, 17a abstützt, in eine vertikale Lage aufgerichtet werden, d.h. um 90° gedreht werden, so wird ein Antriebsmotor 14 eingeschaltet, der die Welle 15 und damit die Antriebsräder 13, 13a dreht. Dabei bewegt sich das Tischgestell 2 mit dem Kipptisch 1 im Uhrzeigersinn und die Führungszapfen 9, 9a verlassen die Führungsschienen 7, 7a. Bei dieser Kippbewegung ruht das Tischgestell 2 demgemäss dann nur noch mit den Führungsschienen 8, 8a auf den Führungszapfen 10, 10a und mit den Tragbahnen 17, 17a auf den Tragrollen 16, 16a. Durch die Führung der Führungszapfen 10, 10a in den Führungsschienen 8, 8a wird erreicht, dass der Kipptisch 1 in die in der Fig. 2 dargestellte Lage aufgerichtet werden kann, ohne dass er mit seinem unteren Ende am Boden anstösst.

Soll der Kipptisch 1 aus seiner in der Fig. 1 dargestellten Horizontallage entgegen dem Uhrzeigersinn verschwenkt werden, so verlassen in analoger Weise die Führungszapfen 10, 10a die Führungsschienen 8, 8a und die Führung erfolgt durch die Führungszapfen 9, 9a in den Führungsschienen 7, 7a. Die Antriebsräder 13, 13a drehen sich dabei entgegengesetzt zu ihrer Drehung bei der Aufrichtung in die Lage gemäss Fig. 2. Das Tischgestell 2 wird dabei mit dem Kipptisch 1 entgegen dem Uhrzeigersinn in die in der Fig. 3 dargestellte Kopf-Tief-Lage verschwenkt, in der der Kipptisch 1 mit dem Boden einen Winkel von etwa 30° einschliesst.

Beim Verstellen des Kipptisches 1 aus der Horizontallage gemäss Fig. 1 in die Endlagen gemäss den Figuren 2 und 3 verlässt demgemäss jeweils einer der Führungszapfen 9, 9a, 10, 10a seine Führungsbahn 7, 7a, 8, 8a, und das jeweils bodennähere Ende des Gerätes wird so weit angehoben, dass es über dem Bodenniveau liegt, wobei das Tischgestell 2 von den Tragrollen 16, 16a auf den Tragbahnen 17, 17a und den jeweiligen Führungszapfen 9, 9a, 10, 10a in den Führungsschienen 7, 7a, 8, 8a getragen wird.

Um Gleitreibung in den Führungsschienen 7, 7a, 8, 8a zu vermeiden, tragen die Führungszapfen 9, 9a, 10, 10a zweckmässigerweise Wälzlager. Für eine ruckfreie, sichere Bewegung des Tischgestelles 2 kann jede der Antriebsbahnen 12, 12a auch aus mehreren parallelen Ketten bestehen, in die jeweils ein Antriebsrad auf der Welle 15 eingreift. Es ist zweckmässig, in diesem Fall für jede Antriebsbahn drei unmittelbar nebeneinander parallel verlaufende Ketten vorzusehen, wie dies in der Fig. 4 angedeutet ist.

Aus der Fig. 4 geht hervor, dass die Tragbahnen 17, 17a seitlich etwas über das Tischgestell 2 vorstehen und von zu beiden Seiten des Tischgestelles 2 ortsfest angeordneten Sicherungshaken 18, 18a umgriffen werden, die ein unbeabsichtigtes Abheben des Tischgestelles 2 von den Tragrollen 13, 13a verhindern. Die Sicherungshaken 18, 18a sowie der Antriebsmotor 14 sind in den Figuren 1 bis 3 der Übersichtlichkeit halber nicht dargestellt.

## Patentansprüche

1. Röntgendiagnostikgerät mit einem Kipptisch (1), der aus der horizontalen Lage nach beiden Seiten kippbar ist, davon nach einer Seite um 90°, und der auf einem Tischgestell (2) befestigt ist, das an seiner Aussenseite mit mindestens einer kreisförmigen Antriebsbahn (12, 12a) versehen ist, in die mindestens ein ortsfestes Antriebsrad (13, 13a) für die Kippung eingreift, wobei mindestens auf einer Seite des Tischgestelles (2) zwei geradlinige Führungsschienen (7, 7a, 8, 8a) angeordnet sind, dadurch gekennzeichnet, dass in die Führungsschienen (7, 7a, 8, 8a) in der Horizontallage des Kipptisches (1) zwei ortsfeste Führungszapfen (9, 9a, 10, 10a) eingreifen, wobei die Länge der Führungsschienen (7, 7a, 8, 8a) und die Anordung der Führungszapfen (9, 9a, 10, 10a) so gewählt ist, dass beim Verstellen des Kipptisches (1) aus der Horizontallage jeweils ein Führungszapfen (9, 9a, 10, 10a) seine Führungsschiene (7, 7a, 8, 8a) verlässt und der andere Führungszapfen (9, 9a, 10, 10a) über seine Führungsschiene (7, 7a, 8, 8a) eine derartige Verstellung des Tischgestelles (2) bewirkt, dass das untere Ende des Tischgestelles so weit angehoben wird, dass es über dem Bodenniveau liegt.

2. Röntgendiagnostikgerät nach Anspruch 1, dadurch gekennzeichnet, dass neben dem Antriebsrad (13, 13a) auf dessen Welle (15) eine Tragrolle (16, 16a) befestigt ist, die auf einer auf dem Tischgestell (2) parallel zur Antriebsbahn (12, 12a) verlaufenden Tragbahn (17, 17a) abrollt.

3. Röntgendiagnostikgerät nach Anspruch 1, dadurch gekennzeichnet, dass symmetrisch zu beiden Geräteseiten je eine Antriebsbahn (12, 12a) ein Antriebsrad (13, 13a), eine Tragbahn (17, 17a), eine Tragrolle (16, 16a), zwei Führungsschienen (7, 7a, 8, 8a) und zwei Führungszapfen (9, 9a, 10, 10a) angeordnet sind.

4. Röntgendiagnostikgerät nach Anspruch 3, dadurch gekennzeichnet, dass die Tragbahnen (17, 17a) seitlich etwas über das Tischgestell (2) vorstehen und von zu beiden Seiten des Tischgestelles (2) ortsfest angeordneten Sicherungshaken (18, 18a) umgriffen werden.

## Claims

1. An X-ray diagnostic apparatus including a tiltable table (1) which can be tilted from a horizontal position towards both sides, and to one side by 90°, and which is secured to a table mount (2) which is provided on its outside with at least one circular driving track (12, 12a) into which at least one fixed driving wheel (13, 13) engages for the tilting operation, where two rectilinear guide rails (7, 7a, 8, 8a) are arranged at least on one side of the table mount (2), characterized in that two stationary guide pins (9, 9a, 10, 10a) engage into the guide rails (7, 7a, 8, 8a) when the tiltable table (1) is in the horizontal position, where the length of the guide rails (7, 7a, 8, 8a) and the arrangement of the guide pins (9, 9a, 10, 10a) is selected to be such that when the tiltable table (1) is adjusted out of the horizontal position one guide pin (9, 9a, 10, 10a) will in each case move out of its guide rail (7, 7a, 8, 8a) and the other guide pin (9, 9a, 10, 10a) will adjust the table mount (2) via its guide rail (7, 7a, 8, 8a) in such manner that the lower end of the table mount is raised to above ground level.

2. An X-ray diagnostic apparatus as claimed in Claim 1, characterized in that beside the driving wheel (13, 13a), a bearing roller (16, 16a) is attached to its shaft (15), which bearing roller rolls along a bearing track (17, 17a) which extends parallel to the driving track (12, 12a) on the table mount (2).

3. An X-ray diagnostic apparatus as claimed in Claim 1, characterized in that a driving track (12, 12a), a driving wheel (13, 13a), a bearing track (17, 17a), a bearing roller (16, 16a), two guide rails (7, 7a, 8, 8a) and two guide pins (9, 9a, 10, 10a) are arranged symmetrically on each side of the apparatus.

4. An X-ray diagnostic apparatus as claimed in Claim 3, characterized in that the bearing tracks (17, 17a) laterally project somewhat beyond the table mount (2) and are gripped by fixed securing hooks (18, 18a) arranged on both sides of the table mount (2).

## Revendications

1. Appareil de radiodiagnostic comportant une table basculante (1), qui peut basculer des deux côtés à partir de la position horizontale et ce de 90° d'un côté, et qui est fixée sur un bâti de table (2), qui est équipé, sur sa face extérieure, d'au moins une piste circulaire d'entraînement (12, 12a) dans laquelle s'engage au moins une roue fixe d'entraînement (13, 13a) servant à réaliser le basculement, des rails rectilignes de guidage (7, 7a, 8, 8a) étant disposés au moins d'un côté du bâti (2) de la table, caractérisé par le fait que deux tétons fixes de guidage (9, 9a, 10, 10a) s'engagent dans les rails de guidage (7, 7a, 8, 8a) lorsque la table basculante (1) est dans la position horizontale, auquel cas la longueur des rails de guidage (7, 7a, 8, 8a) et la disposition des tétons de guidage (9, 9a, 10, 10a) est choisie de telle sorte que, lors du déplacement de la table basculante (1) à partir de la position horizontale, un téton respectif de guidage (9, 9a, 10, 10a) quitte son rail de guidage (7, 7a, 8, 8a) et que l'autre téton de guidage (9, 9a, 10, 10a) réalise, par l'intermédiaire de son rail de guidage (7, 7a, 8, 8a) un déplacement tel du bâti (2) de la table que l'extrémité inférieure de ce bâti est soulevée au point qu'elle se situe au-dessus du sol.

2. Appareil de radiodiagnostic suivant la revendication 1, caractérisé par le fait qu'en dehors de la roue d'entraînement (13, 13a) se trouve fixé, sur l'arbre (15) de cette roue, un galet de support (16, 16a) qui roule sur une piste de support (17, 17a), qui, dans le bâti (2) de la table, est parallèle à la piste d'entraînement (12, 12a).

3. Appareil de radiodiagnostic suivant la revendication 1, caractérisé par le fait qu'une piste d'entraînement (12, 12a), une roue d'entraînement (13, 13a), une piste de support (17, 17a), un galet de support (16, 16a), deux rails de guidage (7, 7a, 8, 8a) et deux tétons de guidage (9, 9a, 10, 10a) sont disposés symétriquement des deux côtés de l'appareil.

4. Appareil de radiodiagnostic suivant la revendication 3, caractérisé par le fait que les pistes de support (17, 17a) font légèrement saillie latéralement par rapport au bâti (2) de la table et sont entourées par des crochets de sécurité (18, 18a) montés fixes des deux côtés du bâti (2) de la table.

FIG 1

FIG 2

FIG 3

FIG 4